# EUROPEAN PATENT APPLICATION

(11) **EP 0 570 920 A1**
(43) Date of publication of application: **24.11.1993**
(21) Application number: 93108111.1
(22) Date of filing: 18.05.1993
(51) Int. Cl.: C07D 311/80, C07B 59/00, G01N 33/534, G01N 33/94

(54) **Probe for cannabinoid receptors**

(30) Priority: 19.05.1992 IL 101927
(71) Applicant: YISSUM RESEARCH AND DEVELOPMENT COMPANY OF THE HEBREW UNIVERSITY OF JERUSALEM, Jerusalem 91 999 (IL)
(72) Inventor: Mechoulam, Raphael, Jerusalem (IL); Breuer, Aviva, Jerusalem (IL)
(74) Representative: Weisert, Annekäte, Dipl.-Ing. Dr.-Ing.

(57) **Abstract**

The C₁ -equatorial epimer of 7-hydroxy-hexahydrocannabinol-5'-dimethyl heptyl in an essentially pure stereospecific form, and isotopically tagged derivatives thereof. The preferred tagged compound is the 1,6-ditritiated derivative. The above are novel probes for cannabinoid receptors. A process for the production of these is provided.

## Description

### FIELD OF THE INVENTION:

The invention relates to a novel probe for cannabinoid receptors. These probes are specific compounds, as defined hereinafter, and the invention relates, in preferred aspects, to a certain optically active form of this compound. A still more preferred embodiment of the invention relates to the preferred isomer, in tagged form (the tritiated form of said equatorial epimer). The invention further relates to a process for the production of such epimer and for the production of the tritiated compound.

### BACKGROUND OF THE INVENTION:

A cannabinoid receptor was recently identified in the brain, Devane, W.A. et al., Mol. Pharmacol. 1988, 34, 605-613, followed shortly by reports on its distribution and localization, on its primary structure, on the cloning of the gene involved in its formation, and on the nucleotide sequence of the human receptor cDNA.

(2) Herkenham, M, et al., Proc. Natl. Acad. Sci. USA, 1990, 87, 1932-1936. Lynn, A.B. et al., Neurosceince, 1991, 11,563-583. The radiolabelled probe used in most of these investigations was [³H]1, which, in its non-tritiated form (CP-55940), has a cannobinoid pharmacological profile. Structually, however, 1 is only marginally related to the natural Δ-tetrahydrocannabonol (Δ¹-THC, (2). An additional probe, with a typical THC-type cannabinoid structure, with a K in the picomolar range and with high stereospecificity, both in pharmacological activity and on binding, may help further exploration of this exciting new area of research.

Compound (3) (HU-210) is apparently the most potent cannabimimetic prepared so far. In pharmacological tests, in various animal species, (3) has been shown to be 100-800 times more potent than Δ¹-THC; in drug discrimination in pigeons (3) was 80 times more potent than Δ¹-THC. It completes fully for the specific binding of [³H]1 to membranes from rat brain in heterologous displacement studies (K = 234 pM). Compound (4) (HU-211), the (+) (3S, 4S) enantiomer of (3) was several thousand times less potent than (3) in in vivo pharmacological tests; the potency ratio of 3 to 4 for binding to the cannabinoid receptor is circa 1500. Hence (3) seemed to represent a suitable candidate for labelling.

We have previously shown that hydrogenation of Δ¹ or Δ⁶-THC leads to cannabimimetically active products, the C-1 equatorial epimer (5a) being more active than the axial one (6a), Edery, H. et al., Ann. N.Y. Acad. Sci. 1971, 191, 40-53. The 7-hydroxyhexahydrocannabinols (5b) and (6b) have also been prepared:
(4) Mechoulam, R. et al., J. Med. Chem. 1980, 23, 1068-1072.

Again, the equatorial epimer (5b) was considerably more active in discrimination tests than the axial epimer (6b) and was about 3-17 times more active than Δ¹-THC (depending on the animal used-pigeon or rat - and on the timing of measurement) ibid. On this basis we assumed that hydrogenation, or tritiation in the labelling experiments of (3), would lead to pharmacologically very active compounds, binding well to the receptor. Indeed hydrogenation of (3) over platinum oxide (Adams catalyst) in ethanol at room temperature for 4 hrs (3.4 atm) led to a nearly 1:1 mixture (by GC) of the C-1 epimers (7) and (8) (combined yield 52%) in addition to hydrogenolysed material (5c) and (6c), which was easily separated from the mixture of epimers by column or thin layer chromatography (TLC). It should be pointed out that on TLC (7) and (8) have the same Rf values, but are well separated on gas chromatography (GC).

### SUMMARY OF THE INVENTION :

The present invention relates to ceratin cannabinol derivatives, to tritiated derivatives of these, to the preparation of such derivatives and to an assay for the cannabinoid receptor wherein such compounds are used as probe. More specifically, the invention relates to 7-hydroxy-hexahydrocannabinol-5'- dimethyl heptyl homologue, to a certain specific epimer thereof and to its preparation. The invention further relates to a process for the selective 1,6-tritiation of such compounds and epimers and for the use of these as probes in a cannabinoid receptor assay. Assuming that the epimer (7) would be considerably more potent than the (8) compound, attempts were made by us to effect hydrogenations leading to stereospecific reductions.

Reduction(in ethanol, 140^{o}C, 48 hrs, 17 atm), catalyzed with a 1:1 mixture of Wilkinson's catalyst (tris triphenyl-phosphine rhodium chloride)-
and Kagan's catalyst [2,3-O-isopropylidene-2,3-dihydroxy-1,4-bis diphenylphosphino butane, (+) enantiomer] (+ DIOP),
Kagan, H.B. et al;, J.A.C.S. (1972), 94 , 6429-6433, gave no hydrogenolysed products and led to the desired epimer (7) with 93% regiospecificity (GC). On crystallization (7), mp 80-82^{o}C, was obtained in a 62% yield. The use of the (-) enantiomer of Kagan's reagent did not lead to the axial epimer 8, but also gave mostly the equatorial one (7). The axial epimer (8) was obtained by reduction (in ethanol, 100°C, 34 atm., 48 hrs) with the (-)-binaphthyl based phosphine (BINAP)-Ru (II) dicarboxylate hydrogenation catalyst described by Noyori's group
(6) Noyori, Acc.Chem.Res.1990,23, 345-350,
Traces of hydrogenolysed products were observed. The ratio of the axial (8) to the equatorial epimer (7) was 85:15. Pure 8 could be obtained on crystallization.

The stereochemistry at C-1 of epimers 7 and 8 was determined by comparison of their NMR spectral data with those reported for the pentyl homologs (5b) (C-7 hydroxymethyl moiety equatorial) and (6b) (C-7 hydroxymethyl moiety axial).

The term "essentially pure stereospecific form" is intended to define the epimeric form in as high a degree of purity as can be conveniently obtianed. Generally this term applies to a degree of purity of at least 80 per cent, and preferably above 85 per cent.

The invention also relates to isotopically tagged forms of the equatorial epimer, preferred tagged form being the tritiated form.

While the above described conditions for the preparation of (7) are facile and reproducible, they are not applicable for the synthesis of tritium labelled (7). For safety reasons, work under high pressure with tritium is not desirable. In addition, in initial experiments we found that ethanol is not a good solvent for the tritiation experiments, as it led to [³H]7 with low specific activity, possibly due to proton exchange.

The present invention relates, in a preferred embodiment, to labelled derivatives of (7), and specifically to the tritiated derivative thereof.

Hydrogenation (and tritiation) can be done using Wilkinson's catalyst alone, in ethyl acetate, at 120^{o}C, with hydrogen introduced at atmospheric pressure. Although considerable hydrogenolysis was observed, the hydroyenolysed compounds were found to be much less polar than (7) or (8) and were easily removed by chromatography (on column or TLC). Under these conditions (7) was obtained with 95% chemical purity (GC). On tritiation, under identical conditions, the tritiated product was purified by TLC. Only one band (>99%) was observed on TLC with UV detection as well as on radiochromatography. The specific activity of [³H]7 was 54 Ci/mmol. This material was subsequently used in our binding experiments.

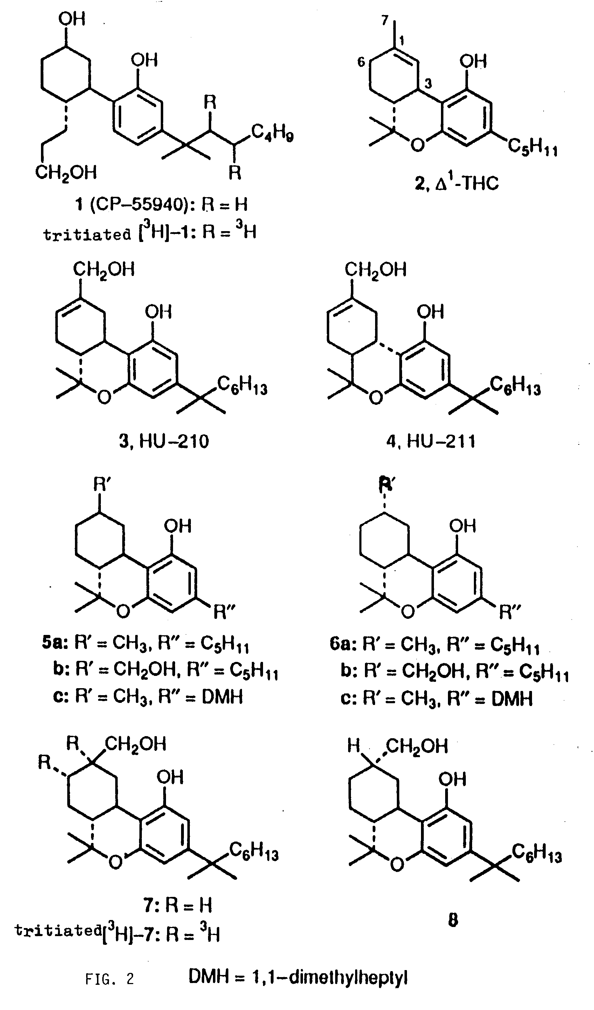

The ability of [³H]7 to bind to the cannabinoid receptor was assessed in a synaptosomal membrane preparation derived from rat whole brain, without brainstem, using a centrifugation assay. Homologous displacement studies indicate that (7) is highly potent at binding to the cannabinoid receptor with a K_{d} of 45 pM (Fig. 2). The binding capacity for these experiments was also high, 4.41 ± 0.08 pmol/mg protein, in agreement with the abundance of cannabinoid receptors previously detected using [³H]CP-55940 and different assay conditions . The ability of other cannabinoids to displace [³H]7 binding was also assessed in heterologous displacement studies. Compound 3 (HU-210) exhibited a Kᵢ of 188 pM, while its (+)-enantiomer, compound (4) (HU-211) was 2500-fold less potent with a Kᵢ of 466 nM. The axial epimer, compound 8, was also potent with a Kᵢ of 190 pM. Δ¹-THC was able to fully compete with [³H]7 for binding to the cannabinoid receptor, but was 1000 fold less potent, with a Kᵢ of 46 nM.

Thus, while in the discrimination tests (3) and (7) are essentially equivalent in activity, in the binding experiments 7 is about 4 times more potent than (3). Compound (7) is about 1000 times more potent than Δ¹-THC on binding, but only 125 times more potent in the in vivo experiment. Such differences are not unexpected, as binding and in vivo pharmacological data are not directly comparable.

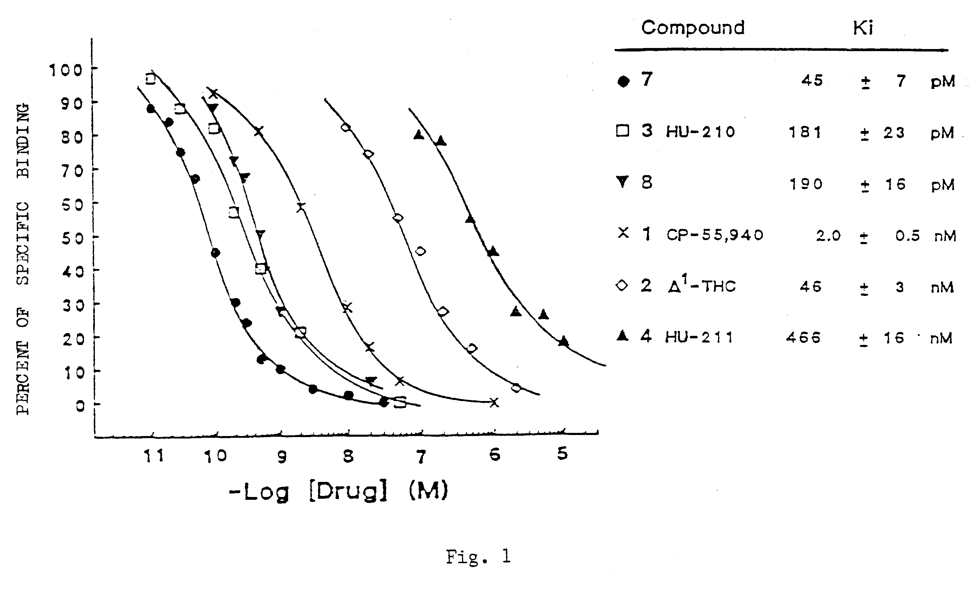

As mentioned above CP-55940 (1) has been widely used for binding (or displacement) on the cannabinoid receptor. We have compared the binding potency of CP-55940 with that of (7) (Fig. 1); the latter compound is more potent than CP-55940 which, under our conditions, has a Kᵢ of 2.0 nM. Compound (7) is thus apparently the most active cannabinoid reported to bind to the cannabinoid receptor.

The results presented above indicate that (7) a potent cannabimimetic compound, which is easily obtained in a tritiated form, can serve as a novel, advantageous highly active probe for the cannabinoid receptor.

### Experimental Section

A. Chemistry. Melting points (mp) were taken in glass capillary tubes with a Thomas-Hoover Uni-Melt apparatus. Infrared spectra (IR) were recorded on a JASCO A-200 spectrophotometer. Nuclear magnetic resonance (NMR) spectra were recorded on a Varian VXR-300 S instrument, at 300 MHz, for ¹H and 75.429 MHz for ¹³C. Rotations were determined on a Perkin-Elmer Model 141 polarimeter in chloroform. The hydrogenations were performed in a stainless steel vessel; the hydrogen was introduced at the pressure indicated before closing the vessel. The microanalyses were performed by the Microanalytical laboratory of the Hebrew University and the elemental compositions of the compounds agreed to within ± 0.4% of the calculated value. High Resolution Mass Spectra (HRMS) were recorded on a Varian 711 at the mass spectrometry center at the Technion, Haifa. Chromatographic separations were performed on silica gel columns (Woelm TSC silica, for dry chromatography, activity III/30 mm, cat. No. 04530). Preparative TLC was performed on Merck, silica gel F₂₅₄, cat No. 5717. Gas chromatography (GC) was performed on a Varian gas chromatograph, model 3700, under the following conditions: injector-270^{o}C: detector-260^{o}C; capillary column fused silica, DB 17, film thickness 0.25 um, column dimensions 30m x 0.245 mm (J.W. Scientific cat.No. 122-1732) at 260^{o}C for the radiochromatography a Berthold scanner, Germany, model LB 2733 was used.
Reduction of 3 with a mixture of Kagan's and Wilkinson's catalysts. A solution of (3) (160 mg, 0.415 mmole), (+)-DIOP (Kagan's catalyst) (10.7 mg, 0.02 mmol) and tris (triphenyl phosphine) rhodium (I) chloride (Wilkinson's catalyst) (19.8 mg, 0.02 mmol) in ethanol (8 ml) was stirred under 13.6 atm of hydrogen at 140^{o} for 48 hrs. The solution was evaporated to dryness and the residue was purified by preparative TLC. The product (140 mg), which showed 1 spot on TLC, consisted of (7) and (8) in a ratio of 93:7 (GC). On crystallization from pentane (7) (100 mg) was obtained, chemical purity > 99% (GC), mp 80-82^{o}C, [α]_{D} -92^{o} (c=10 mg/ml, CHCl₃), ¹H - NMR δ (CDCl₃), 0.85 (t, 3 H w-CH₃), 2.46 (dt, 1H, C-3 H), 3.25 (bd, 1H, C-2 H), 3.52 (d, J=6.3 Hz, 2H,C-7, H), 6.21 (d, 1H, arom), 6.35 (d, 1H, arom). HRMS calcd for C₂₅H₄₀O₃ 388.2978, found 388.3002. Anal. (C₂₅H₄₀O₃) C,H. Acetylation of 7 (10 mg) with acetic anhydride (0.1 ml) and pyridine (0.2 ml), followed by the standard work up, gave the diacetate of (7), an oil which produced (1) spot on TLC and (1) peak on GC, ¹H NMR, δ (CDCl₃) 2.05 (s, 3H, OCOCH₃), 2.29 (s, 3H, OCOCH₃), 2.36 (t, 1H, C-3H) 2.74 (bd, 1H, C-2H), 3.86 (m, 1H, C-7 H), 3.95 (m, 1H, C-7 H), 6.48 (d, 1H, arom) and 6.66 (d, 1H, arom), ¹³C NMR, δ 69.262 ppm (C-7).
Reduction of (3) with Wilkinson's reagent alone. Compound (3) (33 mg) was dissolved in ethyl acetate (4 ml), tris (triphenyl phosphine) rhodium (I) chloride (6 mg) was added and the solution was stirred for 24 hrs, at 120^{o}C, under hydrogen introduced at atmospheric pressure. The reaction mixture was cooled to room temp, filtered and evaporated. It was purified on preparative TLC by elution with ether:petroleum ether (6:4). Two main bands were observed. The less polar band was extracted with ethyl acetate and the solution was evaporated. It consisted of a mixture of 5c and 6c (8 mg), as determined by direct comparison (GC). The more polar band was extracted with ethyl acetate, stirred For 20 min, filtered and evaporated. The residue obtained (20 mg) was a mixture of (7) and (8) in a ratio of 95:5 (GC).

The above reaction conditions were employed for the radiolabelling with tritium. After the yields and level of stereospecificity were first confirmed with unlabelled material, identical conditions were used for tritiation of (3). The tritiated (7) thus obtained was purified by preparative TLC (ether:petroleum ether, 6:4). The material obtained showed one band (>99%) on UV spectrophotometry (ethanol solution) and one band on radiochromatography coincident with that of the reference material (7). The specific activity was 54 Ci/mM, which was determined by dividing the total radioactivity recorded by the number of milimoles of labelled compound. The amount of labelled compound was determined using UV spectroscopy at the 280 nm absorbtion band. On the basis of the "cold" experiment we assume that tritiated (7) has a chemical purity of 95%, the rest being mostly tritiated (8).
Reduction of (3) with Noyori's catalyst. (S)-2,2¹-bis (diphenylphosphino)1,1¹-binaphtyl [(S)-BINAP] (Noyori's catalyst) was prepared as described by Ohta et al,
(Ohta: Inorg. Chem. (1988) 27, 546. This catalyst (10 mg, 0.012 mmole) was added to (3) (286 mg, 0.74 mmole) in ethanol (6 ml). The solution was stirred under 34 atm of hydrogen at 100^{o}C for 48 hr. After evaporation the mixture was purified on preparative TLC using ether:petroleum ether (6:4). Only traces of (5c) and (6c) were noted. The main product obtained (250 mg, 87%) was shown to consist mainly of the axial epimer (8) (ratio of (8) to (7), 85:15, by GC). On crystallization from pentane (8) was obtained, >99% chemical purity (GC), mp 95^{o}C (sintering at 75^{o}C); [α]_{D}-62.3^{o} (8.05 mg/ml CHCl₃); ¹H-NMR, δ (CDCl₃), 0.85 (t, 3 H, w-CH₃), 2.50 (t, 2 H, C-3 H), 3.15 (bd, 2H, C-2H), 3.75 (m, 1H, C-7 H), 3.90 (m, 1H, C-7 H), 6.27 and 6.35 (2d, 2H, arom). HRMS calcd for C₂₅H₄₀O₃ 388.2978, found 388.2970. Anal (C₂₅H₄₀O₃) C, H.
Ligand binding assay. Radiolabelled (7) (ratio of (7) to (8), 95:5) was stored at a concentration of 1 uM (54 uCi/ml) in absolute ethanol at -20^{o}C. Unlabelled drugs were stored as 10 mM and 100 uM ethanol solutions at -20^{o}C. Following evaporation of the ethanol, drug dilutions were performed serially from 1 uM or 100 uM solutions in a vehicle of 5 mg/ml fatty acid free BSA (Sigma) in water. Due to the partial insolubility of Δ¹-THC at concentrations above 5 uM in 5 mg/ml BSA, 0.01 ml of 20 mM Δ¹-THC in ethanol was added directly to 0.99 ml of 50 mg/ml BSA and further dilutions were made such that the vehicle contained 5 mg/ml BSA and 0.1% (v/v) ethanol. The radioligand was placed in a beaker, the ethanol evaporated, and 0.2 ml of 50 mg/ml BSA was added, followed by 100 ml of TME buffer. This was aliquoted (0.8 ml) to siliconized microfuge tubes (Sigma), followed by the addition of 0.1 ml drug or vehicle and 0.1 ml of synaptosomal membrane preparation in TME buffer. The final assay volume of 1 ml contained 45 mM Tris-HCl 2.7 mM MgCl, 0.9 mM EDTA, 0.58 mg BSA, 2.4 to 3.8 ug protein of synaptosomal membrane preparation, 38 to 48 fmol of [³H]7, and vehicle or drug as indicated. The assays tubes For Δ¹-THC also contained 0.01% (v/v) ethanol.

Tubes were incubated at 30^{o}C for 90 min and then centrifuged at 13,000 rpm for 6 min. Following centrifugation, samples of the supernatant were saved and counted. The remaining supernatant was aspirated off and the tubes were placed inverted on drying pins. After 20 min, the tips of the tubes containing the pelleted membranes were cut off with a heated scalpel blade in a rig designed to ensure consistency. The tips were then placed in an emulsifier cocktail (Packard), vortexed and after several hours the radioactivity was determined as DPM. The radioligand adhering to the remaining part of the vial after tip removal was also determined. Nonspecific binding to the microfuge tip was assessed in tubes containing radioligand and protein but which were not centrifuged. This value was subtracted from the total binding of the cut tips to give the total bound to the pelleted membranes. Specific binding was defined as the difference between the total bound to the pelleted membranes in the absence and presence of 50 nM unlabelled (7), and was typically 70 to 80% of the total bound. For example, in a set of tubes containing 39.8 fmol total of [³H] (7), 4.63 fmol was specifically bound to the pelleted membrane, 1.3 fmol was nonspecifically bound to the pelleted membrane, 0.75 fmol adhered to the centrifuge tube tip, 10 fmol adhered to the remaining part of the centrifuge vial, and 23.1 fmol remained in solution. Assays were performed in triplicate and experiments were repeated three times. K_{d} and Kᵢ values were determined using the LIGAND program (version 2.3.11, May 1987),
Munson, et al. Anal. Biochem. 1980, 107, 220,239 and results are given as means ± S.E. The bound values used in the calculations represented the total [³H]7 bound to the the pelleted membrane, whereas the total ligand concentration value used included the total [³H]7 bound to the pelleted membrane, that which adhered to the vial, and that which remained in solution.

## Claims

1. The C₁-equatorial epimer of the compound 7-hydroxy-hexahydrocannabinol-5'-dimethyl heptyl homolog , of the formula : 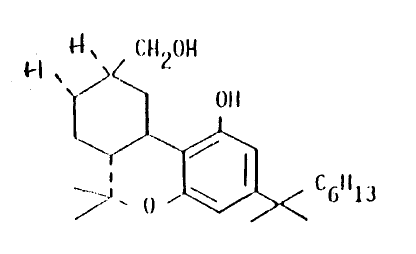 in essentially pure stereospecific forms, and isotopically tagged derivatives thereof.

2. A compound according to claim 1, which is the 1,6-dititriated 7-hydroxy-hexahydrocannabinol-5'-dimethyl heptyl homolog in the form of the at least 85 per cent pure C₁ -equatorial stereospecific epimer.

3. A process for the production of the C₁-equatorial epimer of claim 1 of above 80 % purity, which comprises reducing a compound of Formaula (3) 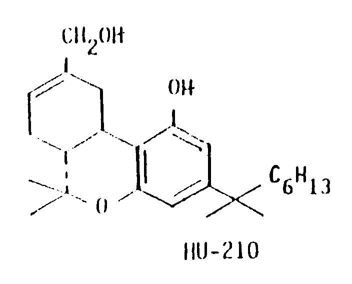 in ethanol at elevated temperature and pressure, catalyzed with a 1:1 mixture of Wilkinson's catalyst (tris triphenyl-phosphine rhodium chloride)-and Kagan's catalyst [2,3-O-isopropylidene-2,3-dihydroxy-1,4-bisdiphenylphosphino butane, (+) enantiomer] (+ DIOP)

4. A process for producing the equatorial epimer defined in claim 1, which comprises hydrogenating the compound of Formula (3) defined in claim 3, in ethylacetate, at elevated temperature, with hydrogen introduced at atmospheric prssure,. catalyzed by Wilkinson's catalyst, and removing hydrogenolyzed products, resulting in a product of above 90 % purity,

5. A process for producing the tritiated derivative claimed in claim 2 , which comprises reducing a compound of Formula (3) 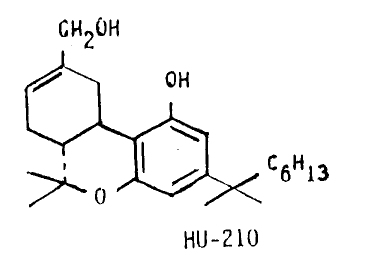 in ethyl acetate, at elevated temperature, with the introduction of tritium, and purifying the resulting product.

6. A product according to claim 5, where the reduction is carried out at about 120^{o}C, and where the purification is by means of TLC.
